# DEMANDE DE BREVET EUROPEEN

(11) **EP 0 714 971 A1**
(43) Date de publication de la demande: **05.06.1996**
(21) Numéro de dépôt: 95402621.7
(22) Date de dépôt: 21.11.1995
(51) Int. Cl.: C10M 135/04, B01J 37/20, C07C 319/00

(54) **Hydrocarbures éthyléniques sulfurés par le soufre élémentaire en présence d'alcanol-amines, leur préparation et leur utilisation**

(30) Priorité: 28.11.1994 FR 9414209
(71) Demandeur: INSTITUT FRANCAIS DU PETROLE, F-92502 Rueil-Malmaison (FR)
(72) Inventeur: Born, Maurice, F-92000 Nanterre (FR); Delfort, Bruno, F-75005 Paris (FR); Lacome, Thierry, F-92500 Rueil Malmaison (FR)

(57) **Abrégé**

On décrit des produits soufrés dérivant d'hydrocarbures mono ou polyéthylèniques par sulfuration au moyen de soufre élémentaire en présence d'au moins une alcanolamine ou de morpholine, et en présence ou en l'absence d'eau.

Les produits soufrés, qui peuvent contenir de 20 à 70 % en masse de soufre, sont généralement solubles dans les lubrifiants, dont ils améliorent les propriétés antiusure et extrême-pression. lls peuvent également être utilisés comme agents de sulfuration, notamment dans la préparation de catalyseurs de raffinage de produits pétroliers.

## Description

L'invention concerne de nouveaux produits organiques soufrés, un procédé pour leur préparation et leurs utilisations.

Divers produits organiques soufrés sont utilisés depuis longtemps comme additifs dans les lubrifiants pour en améliorer notamment des propriétés antiusure et extrême-pression. Il peut s'agir notamment de lubrifiants pour moteurs pour engrenages fortement chargés ou de lubrifiants pour le travail des métaux.

Comme additifs antiusure et extrême-pression ont été largement décrits des produits organiques soufrés obtenus par divers procédés comprenant principalement deux étapes:
1) la formation d'un produit d'addition entre un chlorure de soufre (mono ou di-chlorure de soufre) et un composé à insaturation éthylènique et,
2) la réaction de ce produit d'addition avec un composé sulfuré : sulfure, hydrosulfure ou polysulfure de métal alcalin (par exemple le sodium) mis en jeu tels quels ou formés in situ par réaction d'hydrogène sulfuré (H₂S) ou de mercaptans avec un hydroxyde de métal alcalin (par exemple la soude). Pour accroître la teneur en soufre des produits obtenus il était possible de mettre en jeu du soufre élémentaire au cours de la seconde étape décrite ci-dessus.

Du fait de la mise en jeu de composés chlorés dans la première étape de leur préparation, des produits soufrés de ce type contiennent toujours une certaine quantité résiduelle de chlore qui a pu être réduite à moins de 250 ppm par diverses améliorations apportées au procédé, notamment par la demanderesse, ces dernières années.

De plus, ces procédés engendrent la formation, comme sous produits, de chlorure de métal alcalin (en général NaCl) et de dérivés organiques alcalins hydrosolubles, que l'on retrouve sous la forme d'une solution aqueuse très polluante, produite en quantité très importante et de ce fait très coûteuse à éliminer.

On connaît par ailleurs divers procédés de préparation de produits soufrés ne mettant pas en jeu de composés chlorés. Certains de ces procédés sont basés sur la sulfuration des oléfines par le soufre élémentaire et l'hydrogène sulfuré (H₂S) ; ces procédés présentent l'inconvénient de mettre en jeu un réactif extrêmement toxique, difficile à stocker et à transporter et de développer des pressions généralement élevées, qui peuvent aller par exemple jusqu'à 90 bars.

D'autres procédés mettent en jeu du soufre élémentaire et des hydrosulfures alcalins ou alcalino-terreux.

On a maintenant découvert qu'il était possible de préparer des produits soufrés à partir d'hydrocarbures mono ou polyéthylèniques en utilisant du soufre élémentaire comme seul réactif soufré en opérant en présence d'au moins une alcanolamine ou de morpholine en présence ou en l'absence d'eau.

Les produits soufrés de l'invention sont exempts de chlore, ils présentent en général une teneur en soufre pouvant aller d'environ 20 à 70 % en masse selon l'hydrocarbure éthylénique de départ.

Les produits soufrés de l'invention sont solubles à des degrés divers dans les huiles minérales et dans les polyalphaoléfines hydrogénées, même - ce qui est tout à fait inattendu - les produits ayant une teneur en soufre élevée.

Les produits soufrés de l'invention peuvent être définis d'une manière générale par le fait qu'ils sont obtenus par réaction d'au moins un hydrocarbure mono ou poly éthylénique renfermant en général de 2 à 36 atomes de carbone et de 1 à 3 insaturations éthyléniques avec une quantité appropriée de soufre élémentaire en présence d'au moins une alcanolamine et/ou de morpholine, en présence ou en absence d'eau.

Les hydrocarbures mono ou polyéthylèniques de départ peuvent être à chaîne ouverte, linéaire ou ramifiée, ou cycliques. Comme exemples, on peut citer : l'éthylène, le propylène, le butène-1, les n-butènes-2, l'isobutène, les divers pentènes, les divers hexènes (par exemple le 2,3-diméthyl 1-butène) ainsi que le 1,3 butadiène, l'isoprène, le cyclopentadiène, le dicyclopentadiène, les dimères et trimères de l'isobutène, les trimères et tétramères du propylène, ainsi que les polyalphaoléfines non hydrogénées de faible masse molaire (allant par exemple jusqu'à environ 500). On utilise de préférence l'isobutène.

Les alcanolamines mises en jeu sont principalement les mono-, di- et triéthanolamines. On peut aussi utiliser la morpholine ou des dérivés de la morpholine ainsi que les mélanges des composés précités.

Dans la réaction de préparation des produits soufrés de l'invention, on met en jeu une proportion de soufre élémentaire allant jusqu'à environ 200 g (soit environ 6 moles) par insaturation éthylènique dans l'hydrocarbure de départ.

La proportion d'alcanolamine ou de morpholine par rapport à l'hydrocarbure mono- ou polyéthylènique de départ peut varier dans de larges limites. En particulier, pour l'éthanolamine, utilisée préférentiellement, la proportion peut être de 1 à 250 g par insaturation éthylénique. On remarque avec surprise que l'alcanolamine ou la morpholine peut être utilisée notamment en très faible proportion et qu'on n'en retrouve pas dans le produit soufré finalement obtenu. Aussi on peut penser qu'elle agit essentiellement comme promoteur de la sulfuration.

Dans le cas où l'on met en jeu de l'eau dans le milieu réactionnel, la proportion peut varier largement et aller par exemple jusqu'à 350 g par insaturation éthylènique de l'hydrocarbure de départ.

En général, la réaction est réalisée à une température de 50 à 200 °C et le plus souvent de 90 à160°C.

La pression, qui dépend de manière prépondérante de la nature du ou des hydrocarbures mono ou polyéthylèniques de départ, peut aller de la pression atmosphérique jusqu'à par exemple 50 bars. Ainsi, pour les hydrocarbures éthylèniques gazeux dans les conditions normales, la pression s'établit entre 10 et 50 bars. selon la proportion entre l'hydrocarbure éthylénique et l'alcanolamine (ou la morpholine). La durée de la réaction est par exemple de 0,5 à 24 heures.

Les produits de l'invention sont des produits liquides renfermant généralement de 20 à 70 % en masse de soufre , ils sont limpides et homogènes même aux hautes teneurs en soufre. Leur coloration varie selon la nature de l'hydrocarbure de départ et la quantité de soufre fixée. Ils peuvent être de très peu ou peu colorés (c'est le cas par exemple pour les hydrocarbures monoéthylèniques tels que l'isobutène) à très colorés (dans le cas par exemple des hydrocarbures polyéthylèniques).

Dans le cas des hydrocarbures monoéthylèniques les produits soufrés se caractérisent par la quasi absence d'atomes de carbone éthylèniques. Par ailleurs, les produits soufrés de l'invention sont également exempts d'azote.

Les teneurs en soufre des produits de l'invention leur confèrent des propriétés antiusure et extrême-pression telles qu'ils peuvent être utilisés avantageusement comme additifs dans les huiles et graisses lubrifiantes, notamment dans la formulation d'huiles pour engrenages d'automobiles et d'huiles industrielles.

Pour ces utilisations, on incorpore en général les produits de l'invention aux compositions lubrifiantes à des concentrations de 0,05 à 20 %, de préférence de 0,5 à 10 % en masse.

Certains des produits soufrés de l'invention, notamment ceux qui dérivent de la sulfuration de l'isobutène, peuvent encore être utilisés comme agents de sulfuration en particulier dans la préparation de catalyseurs de raffinage de produits pétroliers.

Les exemples suivants illustrent l'invention.

### Exemple 1

Dans un réacteur de 1 litre permettant d'opérer sous pression, équipé d'un agitateur, d'un système d'introduction de réactifs liquides ou gazeux, ainsi que d'un système de contrôle de la température et de la pression, on introduit 75g (2,34 mole) de soufre, 150 ml de monoéthanolamine et 72g (1,285 mole) d'isobutène. On porte le milieu à la température de 130°C pendant 14 heures. La pression maximale atteinte est de 24 bars. Après retour à la température ambiante, on extrait le milieu avec 150 ml de n-heptane. Après lavage à la soude à 10% en masse, puis lavage à l'eau, on évapore le n-heptane sous pression réduite et on recueille 95g d'un liquide limpide dont la teneur en soufre est de 43% en masse.

### Exemple 2

Dans un réacteur de 1 litre permettant d'opérer sous pression, équipé d'un agitateur, d'un système d'introduction de réactifs liquides ou gazeux, ainsi que d'un système de contrôle de la température et de la pression, on introduit 37,5g (1,17 mole) de soufre, 150 ml de mono éthanolamine et 72g (1,285 mole) d'isobutène. On porte le milieu à la température de 130°C pendant 12 heures. La pression maximale atteinte est de 25 bars. Après retour à la température ambiante, on extrait le milieu avec 150 ml de n-heptane. Après lavage à la soude à 10% en masse, puis lavage à l'eau, on évapore le n-heptane sous pression réduite et on recueille 17g d'un liquide limpide dont la teneur en soufre est de 41,2% en masse.

### Exemple 3

Dans un réacteur de 1 litre permettant d'opérer sous pression, équipé d'un agitateur. d'un système d'introduction de réactifs liquides ou gazeux, ainsi que d'un système de contrôle de la température et de la pression, on introduit 225g (7,03 mole) de soufre, 150 ml de mono éthanolamine et 72g (1,285 mole) d'isobutène. On porte le milieu à la température de 130°C pendant 14 heures. La pression maximale atteinte est de 40 bars. Après retour à la température ambiante, on extrait le milieu avec 150 ml de n-heptane. Après lavage à la soude à 10% en masse, puis lavage à l'eau, on évapore le n-heptane sous pression réduite et on recueille 150 g d'un liquide limpide dont la teneur en soufre est de 54,10 % en masse.

### Exemple 4

Dans un réacteur de 1 litre permettant d'opérer sous pression, équipé d'un agitateur, d'un système d'introduction de réactifs liquides ou gazeux, ainsi que d'un système de contrôle de la température et de la pression, on introduit 75g (2,34 mole) de soufre, 300 ml de monoéthanolamine et 72g (1,285 mole) d'isobutène. On porte le milieu à la température de 130°C pendant 3 heures. La pression maximale atteinte est de 28 bars. Après retour à la température ambiante, on extrait le milieu avec 150 ml de n-heptane. Après lavage à la soude à 10 % en masse, puis lavage à l'eau, on évapore le n-heptane sous pression réduite et on recueille 38g d'un liquide limpide dont la teneur en soufre est de 40 % en masse.

### Exemple 5

Dans un réacteur de 1 litre permettant d'opérer sous pression, équipé d'un agitateur, d'un système d'introduction de réactifs liquides ou gazeux, ainsi que d'un système de contrôle de la température et de la pression, on introduit 75g (2,34 mole) de soufre, 150 ml de morpholine et 72g (1,285 mole) d'isobutène. On porte le milieu à la température de 130°C pendant 5 heures. La pression maximale atteinte est de 12 bars. Après retour à la température ambiante, on extrait le milieu avec 150 ml de n-heptane. Après lavage à la soude à 10% en masse, puis lavage à l'eau, on évapore le n-heptane sous pression réduite et on recueille 120g d'un liquide limpide dont la teneur en soufre est de 41,1% en masse.

### Exemple 6

Dans un réacteur de 1 litre permettant d'opérer sous pression, équipé d'un agitateur, d'un système d'introduction de réactifs liquides ou gazeux, ainsi que d'un système de contrôle de la température et de la pression, on introduit 75g (2,34 mole) de soufre, 75 ml de monoéthanolamine, 75 ml d'eau et 72g (1,285 mole) d'isobutène. On porte le milieu à la température de 130°C pendant 14 heures. La pression maximale atteinte est de 31 bars. Après retour à la température ambiante, on extrait le milieu avec 150 ml de n-heptane. Après lavage à la soude à 10% en masse, puis lavage à l'eau, on évapore le n-heptane sous pression réduite et on recueille 82g d'un liquide limpide dont la teneur en soufre est de 48,6 % en masse.

### Exemple 7

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 mole) de soufre, 1,5 ml de monoéthanolamine, 148,5 g d'eau et 24g (0,428 mole) d'isobutène. On porte le milieu à la température de 140°C pendant 17 heures. La pression maximale atteinte est de 19 bars. Après retour à la température ambiante, on extrait le milieu avec 150 ml de n-heptane. Après lavage à la soude à 10% en masse, puis lavage à l'eau, on évapore le n-heptane sous pression réduite et on recueille 16 g d'un liquide limpide dont la teneur en soufre est de 65,0 % en masse.

### Exemple 8

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 mole) de soufre, 150 ml de diéthanolamine et 72g (1,285 mole) d'isobutène. On porte le milieu à la température de 130°C pendant 9 heures. La pression maximale atteinte est de 28 bars. Après retour à la température ambiante, on extrait le milieu avec 150 ml de n-heptane. Après lavage à la soude à 10% en masse, puis lavage à l'eau, on évapore le n-heptane sous pression réduite et on recueille 95g d'un liquide limpide dont la teneur en soufre est de 38% en masse.

### Exemple 9

Dans un réacteur de 1 litre permettant d'opérer sous pression, équipé d'un agitateur. d'un système d'introduction de réactifs liquides ou gazeux ainsi que d'un système de contrôle de la température et de la pression, on introduit 59g (1,84 mole) de soufre, 150 ml de mono éthanolamine et 68g (1,00 mole) d'isoprène. On porte le milieu à la température de 130°C pendant 12 heures. La pression maximale atteinte est de 6 bars. Après retour à la température ambiante, on extrait le milieu avec 150 ml de n-heptane. Après lavage à la soude à 10% en masse, puis lavage à l'eau, on évapore le n-heptane sous pression réduite et on recueille 46g d'un liquide limpide dont la teneur en soufre est de 34,1% en masse.

### Exemple 10

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 33,6g (1,05 mole) de soufre, 150 ml de monoéthanolamine et 75g (0,56 mole) de dicyclopentadiène. On porte le milieu à la température de 130°C pendant 8 heures. La pression maximale atteinte est de 6 bars. Après retour à la température ambiante, on extrait le milieu avec 150 ml de toluène. Après lavage à la soude à 10% en masse, puis lavage à l'eau, on évapore le toluène sous pression réduite et on recueille 74g d'un liquide limpide dont la teneur en soufre est de 19,3% en masse.

### Exemple 11

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 35,1g (1,1 mole) de soufre, 150 ml de monoéthanolamine et 49,6g (0,59 mole) de 2,3-diméthyl 1-butène. On porte le milieu à la température de 130°C pendant 11 heures. La pression maximale atteinte est de 3 bars. Après retour à la température ambiante, on extrait le milieu avec 150 ml de toluène. Après lavage à la soude à 10% en masse, puis lavage à l'eau, on évapore le toluène sous pression réduite et on recueille 30g d'un liquide limpide dont la teneur en soufre est de 28,4% en masse.

### Exemple 12

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 59,2g (1,85 mole) de soufre, 150 ml de monoéthanolamine, 42g (0,75 mole) d'isobutène et 33g (0,25 mole) de dicyclopentadiène. On porte le milieu à la température de 130°C pendant 7 heures. La pression maximale atteinte est de 19 bars. Après retour à la température ambiante, on extrait le milieu avec 150 ml de toluène. Après lavage à la soude à 10% en masse, puis lavage à l'eau, on évapore le toluène sous pression réduite et on recueille 91g d'un liquide limpide dont la teneur en soufre est de 29,6% en masse.

L'examen par C13 RMN et par analyse élémentaire des produits préparés comme décrit dans les exemples 1 à 8 et 11 indique l'absence d'atomes de carbone éthylèniques et l'absence d'azote pour tous les produits préparés comme décrit dans les exemples de 1 à 12.

### Exemple 13 : Examen de la solubilité des produits de l'invention

Les produits de l'invention préparés comme décrit dans les exemples 1 à 5, 8, 11 et 12 sont examinés pour leur solubilité dans une huile minérale 130 Neutral Solvent et une huile synthétique polyalphaoléfine hydrogénée (PAO 6), à une concentration permettant d'ajuster la teneur en soufre à 2% en masse. Les résultats sont rassemblés dans le tableau I.

### Exemple 14 : Caractérisation des propriétés extrême-pression

Les produits de l'invention préparés comme décrit dans les exemples 4, 5, 8, 11 et 12 sont caractérisés pour leurs propriétés extrême-pression dans une huile minérale 130 Neutral solvent à une concentration permettant d'ajuster la teneur en soufre due à l'additif à 2% en masse. La caractérisation est effectuée au moyen d'une machine 4 billes selon l'essai ASTM D2783. Les résultats sont rassemblés dans le tableau II.

**TABLEAU I**

| PRODUIT DE L'EXEMPLE N° | % Masse ADDITIF/HUILE | % Masse S/HUILE | SOLUBILITE/HUILE MINERALE 130N | SOLUBILITE /HUILE SYNTHETIQUE PAO 6 |
|---|---|---|---|---|
| 1 | 4,65 | 2 | oui | oui |
| 2 | 4,85 | 2 | oui | oui |
| 3 | 3,7 | 2 | oui | oui |
| 4 | 5 | 2 | oui | oui |
| 5 | 4,86 | 2 | oui | non |
| 8 | 5,26 | 2 | oui | oui |
| 11 | 7 | 2 | oui | oui |
| 12 | 6,75 | 2 | oui | oui |

**TABLEAU II**

| PRODUIT DE L'EXEMPLE N° | % Masse ADDITIF/HUILE | % Masse S/HUILE | Essai sur machine 4 billes | |
|---|---|---|---|---|
| | | | Charge de soudure (daN) | Indice charge/usure (daN) |
| 4 | 5 | 2 | 410 | 83 |
| 5 | 4,86 | 2 | 420 | 85 |
| 8 | 5,26 | 2 | 400 | 81 |
| 11 | 7 | 2 | 310 | 56 |
| 12 | 6,75 | 2 | 330 | 60 |

## Revendications

1. Produit soufré caractérisé en ce qu'il est obtenu par réaction d'au moins un hydrocarbure mono ou polyéthylènique renfermant de 2 à 36 atomes de carbone et de 1 à 3 insaturations éthylèniques avec du soufre élémentaire, en présence d'un composé choisi dans le groupe formé par les alcanolamines, la morpholine et ses dérivés, éventuellement en présence d'eau.

2. Produit soufré selon la revendication 1, caractérisé en ce que l'hydrocarbure mono ou polyéthylènique est choisi parmi l'éthylène, le propylène, le butène-1, les n-butènes-2, l'isobutène, les divers pentènes, les divers hexènes, ainsi que le 1,3-butadiène, l'isoprène, le cyclopentadiène, le dicyclopentadiène, les dimères et trimères de l'isobutène, les trimères et tétramères du propylène ainsi que les polyalphaoléfines non hydrogénées de masse molaire allant jusqu'à environ 500.

3. Produit soufré selon la revendication 1 ou 2, caractérisé en ce que ledit hydrocarbure est l'isobutène.

4. Produit soufré selon l'une des revendications 1 à 3, caractérisé en ce que l'on met en jeu une proportion de soufre élémentaire allant jusqu'à environ 200 g (soit environ 6 moles) par insaturation éthylènique dans l'hydrocarbure de départ.

5. Produit soufré selon l'une des revendications 1 à 4, caractérisé en ce que l'on met en jeu une mono, une di, ou une trialcanolamine ou la morpholine.

6. Produit soufré selon des revendications 1 à 5, caractérisé en ce que l'on met en jeu la monoéthanolamine en une proportion de 1 à 250 g par insaturation éthylènique de l'hydrocarbure de départ.

7. Produit soufré selon l'une des revendications 1 à 6, caractérisé en ce que l'on met en jeu de l'eau en une proportion allant jusqu'à 350g par insaturation éthylènique de l'hydrocarbure de départ.

8. Produit soufré selon l'une des revendications 1 à 7, caractérisé en ce que la réaction est réalisée à une température de 50 à 200°C et sous une pression allant de la pression atmosphérique jusqu'à 50 bars.

9. Produit soufré selon l'une des revendications 1 à 8, caractérisé en ce qu'il referme de 20 à 70 % en masse de soufre.

10. Utilisation d'un produit soufré selon l'une des revendications 1 à 9 comme additif dans des compositions lubrifiantes, à une concentration de 0,05 à 20 % en masse.

11. Utilisation d'un produit soufré selon l'une des revendications 1 à 9 comme agent de sulfuration dans la préparation de catalyseurs de raffinage de produits pétroliers.
